# EUROPEAN PATENT SPECIFICATION

(11) **EP 2 350 292 B1**
(45) Date of publication and mention of the grant of the patent: **23.10.2013**
(21) Application number: 08803551.4
(22) Date of filing: 02.09.2008
(51) Int. Cl.: C12N 15/85, C12N 15/866, A01K 67/033

(54) **INSECT-DERIVED PROMOTERS FOR FOREIGN PROTEINS EXPRESSION IN INSECT CELLS**
AUS INSEKTEN STAMMENDE PROMOTOREN ZUR EXPRESSION VON FREMDPROTEINEN IN INSEKTENZELLEN
PROMOTEURS DÉRIVÉS D'INSECTES POUR L'EXPRESSION DE PROTÉINES ÉTRANGÈRES DANS DES CELLULES D'INSECTES

(43) Date of publication of application: 03.08.2011
(73) Proprietor: Alternative Gene Expression, S.L. (Algenex), 28223 Pozuelo de Alarcón - Madrid (ES)
(72) Inventor: GÓMEZ SEBASTIÁN, Silvia, 28223 Pozuelo de Alarcon - Madrid (ES); LÓPEZ VIDAL, Javier, 28223 Pozuelo de Alarcon - Madrid (ES); SÁNCHEZ RAMOS, Ismael, 28223 Pozuelo de Alarcon - Madrid (ES); ALONSO MARTÍ, Covadonga, 28223 Pozuelo de Alarcon - Madrid (ES); MARTÍNEZ ESCRIBANO, José, Angel, 28223 Pozuelo de Alarcon - Madrid (ES)
(74) Representative: HOFFMANN EITLE
(86) International application number: PCT/EP2008/061580
(87) International publication number: WO 2010/025764

(56) References cited:
- US-A1- 2005 054 569
- DATABASE EMBL [Online] 29 December 1995 (1995-12-29), "Trichoplusia ni basic juvenile hormone sensitive hemolymph protein (BJHSP2) gene, promoter region." XP002517034 retrieved from EBI accession no. EMBL:U41640 Database accession no. U41640 -& DATABASE EMBL [Online] 29 December 1995 (1995-12-29), "Trichoplusia ni basic juvenile hormone sensitive hemolymph protein (BJHSP1) gene, promoter region." XP002517035 retrieved from EBI accession no. EMBL:U41639 Database accession no. U41639 -& JONES G ET AL: "Identification of regulatory sequences of juvenile hormone-sensitive and -insensitive serum protein-encoding genes" GENE, ELSEVIER, AMSTERDAM, NL, vol. 173, no. 2, 16 September 1996 (1996-09-16), pages 209-214, XP004043217 ISSN: 0378-1119
- DATABASE EMBL [Online] 9 June 1994 (1994-06-09), "Trichoplusia ni clone BJHSP1 hexamerin gene, 5' flanking sequence." XP002517036 retrieved from EBI accession no. EMBL:U09672 Database accession no. U09672
- DATABASE EMBL [Online] 9 June 1994 (1994-06-09), "Trichoplusia ni clone BJHSP2 hexamerin gene, 5' flanking sequence." XP002517037 retrieved from EBI accession no. EMBL:U09673 Database accession no. U09673
- JINWAL U K ET AL: "Sex-, stage- and tissue-specific regulation by a mosquito hexamerin promoter" INSECT MOLECULAR BIOLOGY, vol. 15, no. 3, June 2006 (2006-06), pages 301-311, XP002515941 ISSN: 0962-1075
- MOREIRA C K ET AL: "Primary characterization and basal promoter activity of two hexamerin genes of Musca domestica." JOURNAL OF INSECT SCIENCE (ONLINE) 2004, vol. 4, 2004, page 2, XP002515942 ISSN: 1536-2442
- JONES G ET AL: "Overview of the regulation of metamorphosis-associated genes in Trichoplusia ni." ARCHIVES OF INSECT BIOCHEMISTRY AND PHYSIOLOGY 1996, vol. 32, no. 3-4, 1996, pages 429-437, XP002515943 ISSN: 0739-4462
- CHA H J ET AL: "MONITORING FOREIGN PROTEIN EXPRESSION UNDER BACULOVIRUS P10 AND POLH PROMOTERS IN INSECT LARVAE" BIOTECHNIQUES, INFORMA LIFE SCIENCES PUBLISHING, WESTBOROUGH, MA, US, vol. 32, no. 5, 1 May 2002 (2002-05-01), page 986,988,990,992, XP001206995 ISSN: 0736-6205
- KRAMER S F ET AL: "Ex vivo monitoring of protein production in baculovirus-infected Trichoplusia ni larvae with a GFP-specific optical probe" BIOTECHNOLOGY AND BIOENGINEERING 20030720 US, vol. 83, no. 2, 20 July 2003 (2003-07-20), pages 241-247, XP002515944 ISSN: 0006-3592

## Description

### FIELD OF THE INVENTION

The present invention may be included in the field of biotechnology. The invention refers to regulatory polynucleotide sequences (such as promoters) derived from hexamerin family genes in insects, and their use for foreign protein expression in insect cells and insect larva, for example using the baculovirus expression vector.

### BACKGROUND OF THE INVENTION

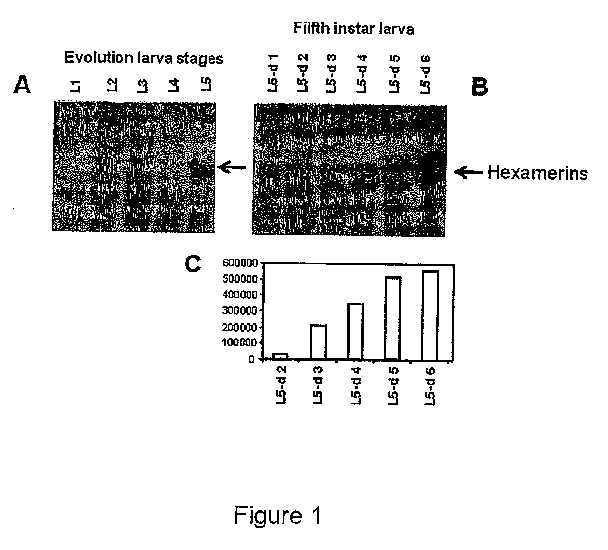

The baculovirus-insect cell expression system is versatile and widely used for producing heterologous (both native and recombinant) proteins. This system is universally recognized as one of the most powerful and versatile to produce recombinant protein of any size. It is based on two main characteristics: the high levels of protein expression achieved and the correct folding of the proteins to become bioactive, while occurring major posttranslational modifications of mammalian cells. In addition, development times of this system are shorter and the genetic stability of the recombinant virus is very high. The baculovirus genome contains two strong promoters called polyhedrin promoter (pL), that is the strongest promoter known among higher organisms, and the promoter p10 (p10 protein). The pL promoter can be used to control the expression of virtually any gene. It is far more rapid and less costly to engineer a baculovirus for protein production than any other advanced production systems based on transformed animal cells or transgenic animals. Nevertheless, previous commercially available promoters have some disadvantages, such as the very late stage in which they promote gene expression. At that time the cellular machinery is largely affected, and then yields of heterologous proteins expressed in insect cells may be impaired, due to incomplete processing and aggregation. These limitations may be due to the deterioration of host cell factors as the viral infection progresses. In order to solve that problem the present invention suggests the use of earlier promoters with stable expression, which are at least as strong as the polyhedrin promoter, as a very interesting option to offset the effects of host cell degradation and, at the same time, to achieve high expression levels of heterologous proteins. The present invention wants to recalls attention to the fact that the transition of the larva (immature) to the adult (mature) form is accomplished by the activation and suppression of a number of genes in various tissues (insect metamorphic development). Among metamorphosis-associated proteins there is a family known as hexamerins that are expressed at very high level during the last instar. These proteins are mostly hemolymph storage proteins which are used during post-larval development. The highest level of the hexamerins can be observed during the first 48 hours of the fifth instar about a four times more after that period (Figure 1). A family of promoters to be used for foreign proteins expression has been developed, departing from the sequences that encode for the above mentioned family of larvae-proteins, in the present invention. Therefore, the present invention provides the promoter pB2 (SEQ ID NO: 2), which is earlier and stronger as compared with promoters used in the state of the Art. The promoters which may drive the expression of major insect proteins at specific evolution larva stages have been isolated in the present invention. They come from the sequence that encodes the proteins hexamerins in larvae and may be used to construct any expression vector (such as baculovirus expression vectors) in order to express heterologous proteins in several insect cells, preferably sf9 or sf21 cells from *Spodoptera frugiperda.* Similar regulatory sequences to those constituting these new promoters had already been characterized in lepidoptera larvae, but never used in the baculovirus expression system before.

### DESCRIPTION OF THE INVENTION

### Brief description of the invention

Thus, the present invention provides regulatory polynucleotide sequences, such as pB2 (SEQ ID NO 2), coming from the sequences that regulate the hexamerin family gene expression. These proteins show the highest expression during the fifth instar, at a very high percentage after the first 48 hours of this larva development period, presenting a very rapid accumulation in a short period of time (Figure 1). These characteristics suggest that the expression of these proteins is driven by strong promoters that could be used to generate more efficient baculovirus or any other expression vectors and, therefore, for optimizing the expression of heterologous proteins. In the case of baculovirus expression systems, an earlier activity of the promoter is especially relevant in case of proteins focalized in secretion pathways, that otherwise could be affected by changes in both cellular post-translational machinery and secretory pathway at very late stages of the baculovirus infection.

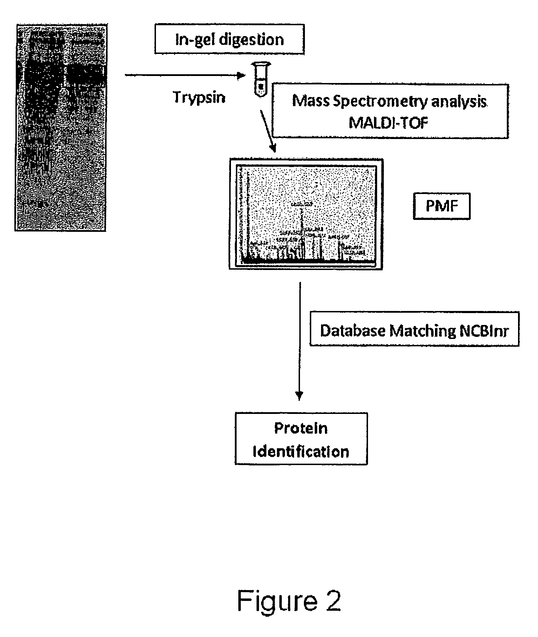

The sequence of the promoter of the invention comes from the sequences that regulate the hexamerin family genes (*BJHSP1 and BJHSP2*) expression, but having some mutations not comprised in the original regulatory nucleotide sequences of said hexamerin family genes. Said hexamerins were isolated form *Trichoplusia ni (T. ni)* larvae and identified in a band of 80 KDa by mass spectrometry analysis (Figure 2). Once proteins were identified, the genes encoding for them were isolated as well as the sequences upstream containing their respective promoters. The primers used to amplify each original promoter are specified in this patent. In a reference aspect, primers characterized by the SEQ ID NO: 3 and 4 were used for amplifying the original promoter which finally derives in the promoter pB1 (SEQ ID NO: 1). Primers characterized by the SEQ ID NO: 5 and 6 were used for amplifying the original promoter which finally derives in the promoter pB2.

The main advantage of the promoter of the invention, specifically pB2, is that it produces an earlier and stronger expression of the protein of interest when compared with the known very late promoters used nowadays in baculovirus expression systems.

In a preferred embodiment, the present invention also includes the combination of the promoter of the invention pB2 with any other promoter, for example pL or p10 (preferably pL). Surprisingly, when such a combination was carried out a synergetic effect was achieved. When a baculovirus expression vector comprising a pLpB2 promoter was tested, the expression levels of the proteins of interest were increased up to 61%-375% (depending on the time of infection) with respect to the use of pL promoter alone. This embodiment is very important for the purpose of the present invention because the promoter pL is, nowadays, generally used in the biotechnology industry.

In other preferred embodiment, the present disclosure also refers to the combination of the promoter of the invention (pB1 and pB2) thereof: pB1-pB1, pB2-pB2 and pB1-pB2. No activity of the promoters of the invention was detected in mammalian cells. Then, the present invention also provides two safety promoters for development of recombinant baculoviruses.

Therefore, the present disclosure elucidates (see the detailed description of the invention) that regulatory polynucleotide sequences (promoters), more effective than those comprised in the state of the Art, could be derived from any polynucleotide sequence located upstream of the open reading frames of the genes that encode for hexamerin family proteins. Therefore, any regulatory polynucleotide sequence derived from any polynucleotide sequence located upstream of the open reading frames of the genes that encode for hexamerin family proteins may be included in the aim of the present disclosure.

For the only purpose of present invention the following terms are defined:
- Regulatory polynucleotide sequence: is a region of DNA generally located upstream (towards the 5' region of the sense strand) of a gene that allows transcription of the gene.
- Vector expression: "vehicle" used to transfer exogenous genetic material inside a cell. This vector contains the necessary regulatory sequences to drive the transcription and translation of a cloned gene or genes and thus transcribe and clone DNA. Baculovirus expression vectors were preferably used in the present invention.
- Baculovirus: is a family of infectious viruses for invertebrates, mainly infecting insects and arthropods.
- Recombinant DNA: It's a form of artificial DNA that is engineered through the combination or insertion of one or more DNA strands, thereby combining DNA that would not normally occur together. Recombinant DNA is produced through the addition of relevant DNA into an existing organism genome.
- Recombinant proteins: Protein encoded by a recombinant DNA.
- Early expression: This term refers to the time at which the promoters begin to express the proteins of interest. In the present invention, this time starting at 16 hours post infection.
- Stronger expression: This term refers to the expression of any protein of interest at equal or higher levels as compared with the level of expression achieved by conventional baculovirus promoters p10 or pL.
- Protein of interest: proteins that can be used for the benefit of humans and animals and that may have industrial, commercial or therapeutic application.
- Biofactory: Cells or organisms genetically modified or not, which can produce proteins at an industrial scale ratio with a number of applications including therapeutic purposes (inhibitors, enzymes, antibodies, antigens, etc.) or primary or secondary products of commercial interest.
- Partial fragment: this expression refers to any partial fragment derived from the regulatory polynucleotide sequences of the invention, having the same improved properties: earlier and stronger as compared with those comprised in the Art.

### Description of the figures

**Figure 1**: shows the absence of an 80 kDa band, which will be identified as hexamerins below, in the first four evolution stages of the *Trichoplusia ni* larva (A) and its presence after 2 days in the fifth larval instar (B). Panel C shows the densitometry quantification of this band during the 6 first days of fifth larval instar.
**Figure 2**: Scheme of mass spectrometry analysis identification of the band of 80 KDa.
**Figure 3**: Expression vectors generated carrying the promoter sequences pB1 or pB2 alone or pB2 in conjunction with the pL sequence (pLpB2).
**Figure 4****:** Insect sf21 cells and insect larvae expressing the green fluorescent protein (GFP) under the control of different promoters (pB1, pB2, pL and pLpB2) after infection with recombinant baculoviruses at different times. In the figure are depicted larvae after 72h of infection.
**Figure 5****:** Expression of GFP by the use of different promoters inserted in baculovirus vectors (pB1, pB2, pL and pLpB2) at different postinfection times (16-72h) and detected by Western blot (A). Recombinant protein was quantified by densitometry of the GFP bands (B) or by fluorometry of cell extracts (C).

### Detailed description of the invention

The present invention provides regulatory polynucleotide sequences, such as the promoters pB2 and in a reference aspect pB1 characterized, respectively, by the SEQ ID NO:2 and the SEQ ID NO: 1 and derived from polynucleotide sequences located upstream of the open reading frames of the genes that encode for hexamerin family proteins in insect larvae. The present invention further refers to the use of such a regulatory polynucleotide sequences for constructing insect expression vectors such as Baculovirus and for expressing heterologous proteins in cells, preferably insect cells. Sequences of the promoters pB1 and pB2 come from the *T*.*ni* hexamerins BJHSP1 and BJHSP2 respectively, which are expressed at high level during the fifth instar and with very high transcriptional activity during the first 48h of this period (Figure 1).

The pB1 sequence is comprised by 1057 nucleotides (SEQ ID NO 1) and derived from the 5' region upstream the initiation codon of the gene encoding the BJHSP1 protein which is the Basic protein 1 suppressible by juvenile hormone. The sequence was amplified from the T.ni genomic DNA by using two specific primers: SEQ ID NO: 3 and SEQ ID NO: 4. The fragment was then cloned in the pGEM-Teasy commercial vector (Promega).

The pB2 sequence is comprised by 1041 nucleotides (SEQ ID NO 2) and derived from the 5'region upstream the initiation codon of the gene encoding the BJHSP2 protein which is the Basic protein 2 suppressible by juvenile hormone. The sequence was amplified from the *T*.*ni* genomic DNA by using two specific primers: SEQ ID NO: 5 and SEQ ID NO: 6. The fragment was then cloned in the pGEM-Teasy commercial vector (Promega).

The pB1 and pB2 sequences were extracted from pGEM-Teasy with *BamH*I and *Sph*I enzymes and cloned into those sites in the pFasTBac dual commercial plasmid (INVITROGEN). This oFasTBac dual vector opened with the mentioned enzymes has the baculovirus promoter sequences eliminated (the vectors obtained were named as: pFBpB1 and pFBpB2). All of them present a cloning site to introduce the cDNA of interest. These were used to generate the recombinant baculoviruses that are able to infect and replicate in insect cells and insect larvae. Additionally, pB2 sequence was extracted by digestion with the restriction enzymes *Not*I *and Pst*I (both restriction sites coming from the pGemT easy vector) and cloned into the same sites in the pFasTBac1 commercial vector (INVITROGEN), after the pL promoter sequence. The vector obtained was named as: pFBpLpB2.

Therefore, the first embodiment of the present invention refers to a method for making an expression regulatory polynucleotide sequence SEQ ID NO: 2 (pB2). Said method comprises the isolation of the SEQ ID NO: 2 (pB2) sequence located upstream of the open reading frames of the genes that encode for the hexamerin family proteins. Once the regulatory polynucleotide sequences were sequenced, they were amplified by conventional means.

In a preferred embodiment of the invention the upstream polynucleotide sequence of hexamerin open reading frames belongs to an insect, preferably *Trichoplusia ni.*

The second embodiment of the present invention refers to a regulatory polynucleotide sequence SEQ ID NO: 2 (pB2), characterized by comprising any sequence derived from any regulatory nucleotide region located upstream of the open reading frames of the genes that encode for the hexamerin family proteins. These promoter sequences present regulatory DNA motifs involved in the modulation of transcriptional activity. Therefore, these sequences can be regulated by hormones during larva development but other signals and also potentially in cell cultures in the context of expression vectors.

In a preferred embodiment the regulatory polynucleotide sequence of the invention: SEQ ID NO: 2 (pB2) is combined with any other regulatory polynucleotide sequence, preferably the promoter pL (promoter of the protein polyedrin) or p10 (promoter of the protein p10) giving rise to different combinations: pLpB2, and p10pB2. The invention provides a very strong promoter sequence when the pL sequence (polyedrin promoter) is combined with the pB2 sequence.

In other preferred embodiment the regulatory polynucleotide sequence of the invention: SEQ ID NO: 2 (pB2) is combined giving rise to the combination pB2pB2 (at least twice repeated). It has been shown that the tandemly located promoters increase efficiency of recombinant protein expression in bacteria and plants (II'ichev-AA et al., Genetika. 1987 23:197-201; Kay R et al., Science. 1987 Jun 5;236(4806):1299-1302.).

The third embodiment of the present invention refers to the use of any of the above mentioned regulatory polynucleotide sequences for constructing expression vectors, preferably a baculovirus or a plasmid.

The fourth embodiment of the invention refers to expression vectors characterized by comprising any of the above explained regulatory polynucleotide sequences and at least a sequence encoding for a protein of interest. In a preferred embodiment the vector is a baculovirus or a plasmid.

The fifth embodiment of the present invention refers to cells transformed, transfected by the expression vector or infected with a recombinant virus used as a vector as described in the previous paragraph. In a preferred embodiment the cells are insect cells preferably sf9 or sf21 from *Spodoptera frugiperda.*

The sixth embodiment of the present invention refers to insect larvae transformed, transfected or infected with the above mentioned expression vectors.

The seventh embodiment of the present invention refers to a method for producing recombinant proteins in insect cells or insect larvae with the above mentioned expression vectors and the extraction and purification of the recombinant protein of interest by conventional means.

The eight embodiment of the present invention refers to the use of said expression vector for producing recombinant proteins.

The ninth embodiment of the present invention refers to the use of said cells for producing recombinant proteins.

The last embodiment of the preset invention refers to the use of said insect larvae as a biofactory for producing recombinant proteins.

### Deposit of microorganisms according to the Budapest treaty

Plasmids were deposited in the Spanish Type Culture Collection (CECT); University of Valencia, Spain with the accession number CECT 7431, on the date July 2, 2008.

### EXAMPLES

This invention is best illustrated by the following examples, which should not be interpreted to be a limitation imposed on the scope thereof On the contrary, it must be clearly understood that other embodiments, modifications and equivalents thereof may be used which this description may suggest to those skilled in the Art without moving away from the spirit of this invention and/or the scope of the attached claims.

The examples of the present invention show that the protein expression takes place at an earlier post-infection time as compared when conventional promoters were used. Moreover, in case of the pB2 and pLpB2 promoter sequences, the expression levels were higher as compared with those achieved in the state of the Art (promoter pL) at both early and late post-infection times.

### Example 1: the green fluorescence protein (GFP) is expressed by the insect promoters of the invention.

### Cloning of the GFP

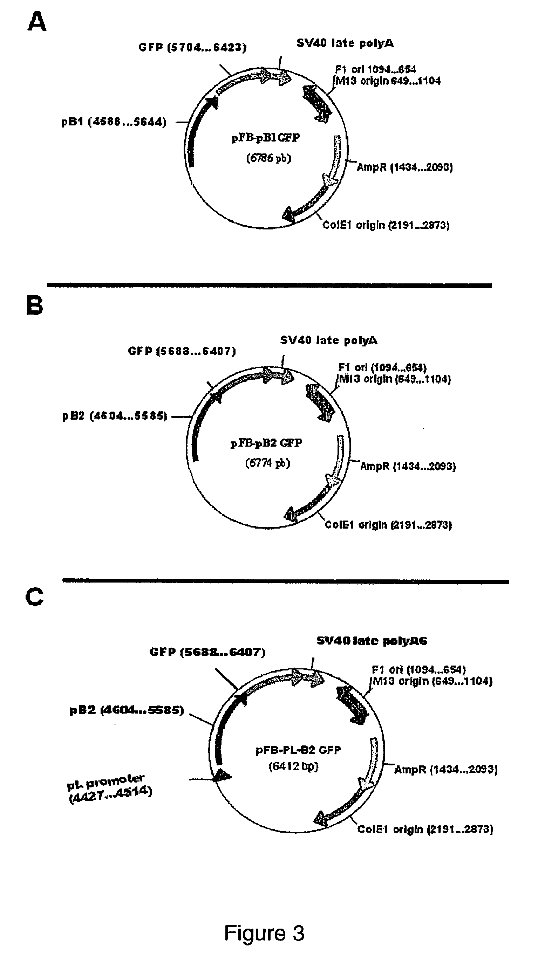

The GFP extracted from the pFBpLGFP plasmid by the *Sph*I restriction enzyme was cloned into the *Sph*I restriction site of the pFBpB1 or pFBpB2 plasmids. The donor-expression plasmids obtained were denominated pFBpB1GFP and pFBpB2GFP respectively. In all the cases the pL sequence was eliminated, so the GFP expression was only due to the activity of the different insect promoters by themselves (Figure 3).

In the other hand, the pB2 promoter flanked by the *Not*I *and Pst*I restriction sites were ligated into the pFBpLGFP also opened by the same restriction enzymes. The donor-expression plasmids obtained were denominated pFBpLpB2GFP and contained the GFP encoding gene under the control of the double promoter pL and pB2 (Figure 3).

### Construction of the GFP-Baculovirus expression vectors

The resulting plasmids were characterized by automated sequencing and used to generate the recombinant baculovirus *BacpB2GFP, BacpB1GFP and BacpLpB2GFP* using the Bac-to-Bac® Baculovirus expression system (Invitrogen, USA) following the manufacture's instructions. Briefly, the resulting donor-expression plasmids were used to transfer DH10Bac™ *E*. *coli* cells for the transposition into the recombinant bacmid. One µg of each bacmid was used to transfect 1 x 10⁶ *Spodoptera frugiperda* sf21 cells using Celifectin® Reagent. The P1 viral stock was obtained after 72 h of incubation at 27°C. The baculovirus was amplified following the manufacturer's instructions. The sf21 insect cells were grown on BD BaculoGold™ TNM-FH Insect Medium (BD Biosciences) supplemented with 50 mg/mL gentamycin. The baculovirus *BacpLGFP,* previously generated in our laboratory, was obtained following identical protocol by using pFBpLGFP plasmid.

### Insect cell inoculation

Cells were then infected at a multiplicity of infection of 0,1 pfu and cells were pelleted at 16h, 24h, 48h or 72h post-infection by centrifugation at 2000 *rpm* for 5 min.

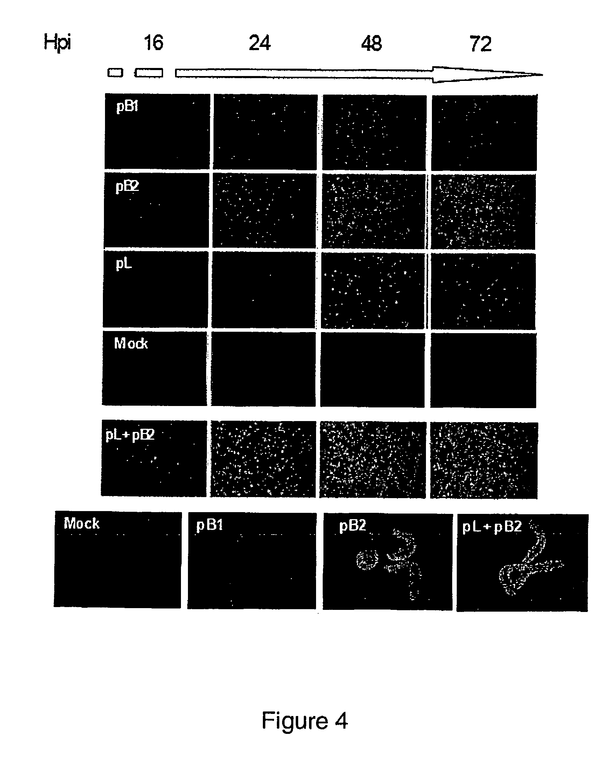

The infected cells were also photographed at different times post-infection in a fluorescence microscope and the pictures are shown in Figure 4. It is remarkable the earlier (starting at 16 h post-infection) activity of the pB2 and pLpB2 promoter sequences and also the strength of both when comparing with the pL promoter, even at late stages of the infection (48 h and 72 h post-infection). The pB1 promoter presents also activity although either not as strong as the pB2 or pL promoters.

### Insect growth conditions and inoculation

Fifth-instar *Trichoplusia ni* larvae were injected with the recombinant baculoviruses near the proleg (forward the body cavity) using 10⁴-10⁵ pfu/larva dose. Infected larvae were kept in growth chambers at 28°C and collected at 72h. Lower panel of Figure 4 shows that pB1 (low), pB2 and the combination of pL and pB2 promoters present activity in insect larvae by expressing the GFP protein in the larvae tissues.

### Preparation of protein extracts

Pelleted cells from a P6 well plate (2x10⁶ cells/well were infected at 0,1 pfu/well) were resuspended in 30 µl of 150 mM NaCl, 1% NP-40, 0,1 % SDS, 50 mM Tris pH 8, protease inhibitors (complete®, Roche), 2-mercaptoethanol (RIPA buffer), keeped on ice for 30 min and clarified by centrifugation at 2000 g for 5 min at 4°C to remove cellular debris.

### Analysis of protein extracts

40 µg of total soluble proteins (TSP) were separated on 12% SDS (w/v) polyacrylamide gels. Proteins were stained with Coomassie Brilliant Blue G-250 (BioRad) or transferred onto nitrocellulose membranes (Hybond C, GE Healthcare), blocked overnight in PBS with 0.1% (v/v) Tween 20 (PBS-T) and 4% skimmed milk (PBS-TM), and incubated for 1 h with GFP primary antibody (Chemicon) diluted 1:1000 and Actine primary antibody (Sigma) diluted 1:500 in PBS-TM. After 2 washes of 10 min in PBS-T, membranes were incubated for 1 h with a 1:2000 dilution of peroxidase-conjugated anti-mouse IgG or anti-rabbit IgG (GE Healthcare) diluted in PBS-TM. After washing, the specific signal was detected using the Advanced ECL system (GE Healthcare) according to the manufacturer's instructions.

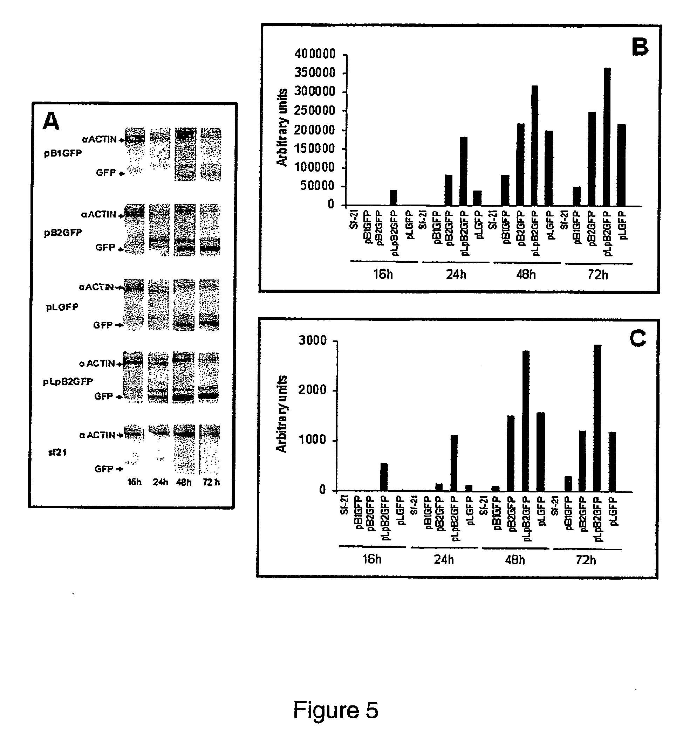

Analysis performed by Western blot yielded the results shown in Figure 5. Again it was demonstrated that pB2 and pLpB2 promoters showed an earlier and stronger activity than pL promoter (figure 5). Densitometry of the specific bands of GFP was measured by using the TINA 2.0 program and using the actine band to normalize the results.

### Fluorimetry assays

The emission of 40 µg of TSP was measured at 535 nm after being excited at 485 nm using a GENIOS fluorimeter. Each value was taken by measuring three times each extract. Results are shown in Figure 5C. Again a synergistic effect of the pL sequence placed 5'upstream the pB2 promoter was clearly observed, with increases of protein production ranging from 61 to 375% depending of the time postinfection with respect to pL promoter alone (Figure 5).

## Claims

1. Method for making expression regulatory polynucleotide sequences that comprises:
a) Isolation and amplification of the SEQ ID NO: 2 (pB2), located upstream of the open reading frames of the genes that encode for the hexamerin family proteins by conventional means.
b) Combination of the SEQ ID NO: 2 (pB2) with the promoter of the protein polyhedrin (pL).

2. Method, according to claim 1, **characterized in that** the amplification of the SEQ ID NO: 2 (pB2) is carried out by using the primers SEQ ID NO: 5 and SEQ ID NO: 6.

3. Method, according to claim 1, **characterized in that** the regulatory polynucleotide sequences belong to an insect larvae, preferably *Trichoplusia ni.*

4. Regulatory polynucleotide sequence **characterized by** comprising the sequence of pB2 (SEQ ID NO: 2) and the sequence of the promoter of the protein polyhedrin (pL).

5. Regulatory polynucleotide sequence, according to the claim 4, **characterized by** comprising the sequence of pB2 (SEQ ID NO: 2) at least twice repeated.

6. Use of the regulatory polynucleotide sequences of claims 4 or 5 for constructing expression vectors, wherein the expression vector is preferably a plasmid or a virus, and more preferably a baculovirus.

7. Expression vector **characterized by** comprising the regulatory polynucleotide sequences of claims 4 or 5 and at least a sequence encoding for a protein of interest.

8. Expression vector, according to claim 7, **characterized in that** it is a virus, preferably a baculovirus, or a plasmid.

9. Expression vector, which is a baculovirus, comprising the sequence of pB2 (SEQ ID NO: 2) and at least a sequence encoding for a protein of interest.

10. Cells transformed or transfected by the expression vector of any of the claims 7-9.

11. Cells, according to the claim 10, **characterized in that** they are insect cells preferably sf9 or sf21 from *Spodoptera frugiperda.*

12. Insect larvae transfected, infected or transformed with the expression vector of any of the claims 7-9.

13. Method for producing recombinant proteins that comprises the inoculation or transfection of the insect cells or insect larvae with the expression vector of any of the claims 7-9 and the extraction and purification of the recombinant protein of interest by conventional means.

14. Use of the expression vector of any of the claims 7-9 for producing recombinant proteins.

15. Use of the cells of claims 10 or 11 for producing recombinant proteins.

16. Use of the insect larvae of claim 12 as a bio factory for producing recombinant proteins.

## Patentansprüche

1. Verfahren zur Herstellung expressionsregulatorischer Polynukleotidsequenzen, umfassend:
a) Isolierung und Amplifizierung der SEQ ID NO: 2 (pB2), welche vor den offenen Leserastern der Gene liegt, die für die Proteine der Hexamerinfamilie kodieren, durch konventionelle Verfahren;
b) Kombination der SEQ ID NO: 2 (pB2) mit dem Promotor des Polyhedrinproteins (pL).

2. Verfahren gemäß Anspruch 1, wobei die Amplifizierung der SEQ ID NO: 2 (pB2) mithilfe der Primer SEQ ID NO: 5 und SEQ ID NO: 6 durchgeführt wird.

3. Verfahren gemäß Anspruch 1, wobei die regulatorischen Polynukleotidsequenzen zu einer Insektenlarve gehören, bevorzugt *Trichoplusia ni.*

4. Regulatorische Polynukleotidsequenz, umfassend die pB2 Sequenz (SEQ ID NO: 2) und die Promotorsequenz des Polyhedrinproteins (pL).

5. Regulatorische Polynukleotidsequenz gemäß Anspruch 4, welche die pB2 Sequenz (SEQ ID NO: 2) mindestens zweimal wiederholt umfasst.

6. Verwendung der regulatorischen Polynukleotidsequenzen gemäß dem Anspruch 4 oder 5 zur Herstellung von Expressionsvektoren, wobei der Expressionsvektor bevorzugt ein Plasmid oder ein Virus ist und noch bevorzugter ein Baculovirus.

7. Expressionsvektor, welcher die regulatorischen Polynukleotidsequenzen gemäß dem Anspruch 4 oder 5 und mindestens eine Sequenz, die für ein Zielprotein kodiert, umfasst.

8. Expressionsvektor gemäß Anspruch 7, welcher ein Virus ist, bevorzugt ein Baculovirus, oder ein Plasmid.

9. Expressionsvektor, welcher ein Baculovirus ist, umfassend die pB2 Sequenz (SEQ ID NO: 2) und mindestens eine Sequenz, die für ein Zielprotein kodiert.

10. Zellen transformiert oder transfiziert mit dem Expressionsvektor gemäß einem der Ansprüche 7-9.

11. Zellen gemäß Anspruch 10, welche Insektenzellen sind, bevorzugt sf9 oder sf21 von *Spodoptera frugiperda.*

12. Insektenlarven transfiziert, infiziert oder transformiert mit dem Expressionsvektor gemäß einem der Ansprüche 7-9.

13. Verfahren zur Herstellung rekombinanter Proteine, welches die Inokulation oder Transfektion von Insektenzellen oder Insektenlarven mit dem Expressionsvektor gemäß einem der Ansprüche 7-9 und die Extraktion und Aufreinigung des rekombinanten Zielproteins durch konventionelle Verfahren umfasst.

14. Verwendung des Expressionsvektors gemäß einem der Ansprüche 7-9 zur Herstellung rekombinanter Proteine.

15. Verwendung der Zellen gemäß Anspruch 10 oder 11 zur Herstellung rekombinanter Proteine.

16. Verwendung der Insektenlarven gemäß Anspruch 12 als Biofabrik zur Herstellung rekombinanter Proteine.

## Revendications

1. Procédé pour fabriquer des séquences polynucléotidiques régulatrices de l'expression, qui comprend :
a) une isolation et une amplification de la SEQ ID NO: 2 (pB2), localisée en amont des cadres de lecture ouverts des gènes qui codent pour les protéines de la famille hexamérine, par un moyen conventionnel.
b) une combinaison de la SEQ ID NO: 2 (pB2) avec le promoteur de la protéine polyhédrine (pL).

2. Procédé selon la revendication 1, **caractérisé en ce que** l'amplification de la SEQ ID NO: 2 (pB2) est réalisée en utilisant les amorces SEQ ID NO: 5 et SEQ ID NO: 6.

3. Procédé selon la revendication 1, **caractérisé en ce que** les séquences polynucléotides régulatrices appartiennent à une larve d'insecte, de préférence *Trichoplusia ni.*

4. Séquence polynucléotidique régulatrice **caractérisée en ce qu'**elle comprend la séquence de pB2 (SEQ ID NO: 2) et la séquence du promoteur de la protéine polyhédrine (pL).

5. Séquence polynucléotidique régulatrice selon la revendication 4, **caractérisée en ce qu'**elle comprend la séquence de pB2 (SEQ ID NO: 2) qui est répétée au moins deux fois.

6. Utilisation des séquences polynucléotidiques régulatrices selon les revendications 4 ou 5 pour construire des vecteurs d'expression, où le vecteur d'expression est de préférence un plasmide ou un virus, et de manière davantage préférée un baculovirus.

7. Vecteur d'expression **caractérisé en ce qu'**il comprend les séquences polynucléotidiques régulatrices selon les revendications 4 ou 5 et au moins une séquence codant pour une protéine d'intérêt.

8. Vecteur d'expression selon la revendication 7, **caractérisé en ce qu'**il est un virus, de préférence un baculovirus, ou un plasmide.

9. Vecteur d'expression, qui est un baculovirus, comprenant la séquence de pB2 (SEQ ID NO: 2) et au moins une séquence codant pour une protéine d'intérêt.

10. Cellules transformées ou transfectées par le vecteur d'expression selon l'une quelconque des revendications 7-9.

11. Cellules selon la revendication 10, **caractérisées en ce qu'**elles sont des cellules d'insecte, de préférence sf9 ou sf21 de *Spodoptera frugiperda.*

12. Larves d'insecte transfectées, infectées ou transformées avec le vecteur d'expression selon l'une quelconque des revendications 7-9.

13. Procédé de production de protéines recombinantes, qui comprend l'inoculation ou la transfection des cellules d'insecte ou des larves d'insecte avec le vecteur d'expression selon l'une quelconque des revendications 7-9, et l'extraction et la purification de la protéine recombinante d'intérêt par un moyen conventionnel.

14. Utilisation du vecteur d'expression selon l'une quelconque des revendications 7-9 pour produire des protéines recombinantes.

15. Utilisation des cellules selon les revendications 10 ou 11 pour produire des protéines recombinantes.

16. Utilisation des larves d'insecte selon la revendication 12 en tant qu'usine biologique pour produire des protéines recombinantes.


### SEQUENCE LISTING

<110> ALGENEX
<120> INSECT-DERIVED PROMOTERS FOR FOREING PROTEINS EXPRESSION IN INSECT CELLS
<130> SEQUENCES
<160> 6
<170> PatentIn version 3.5
<210> SEQ ID NO 1
   <211> 1057
   <212> DNA
   <213> Trichoplusia ni
<220>
   <223> Promotor pB1
<400>
   aaaatgtaca aagattcgca gaattatcaa ttcaacatta aaaagatgca catatatatt   60
   cataatccaa caattttatt acctactgct ggttattgac acaccagcta aggatttatt  120
   gctaactgaa ttcttttgta tactaaataa tttcaccata aaatcggatt gaggaaggaa  180
   aacgtgccaa tcgttatatt tatacatcgg actataggta aacagttata aataatgtat  240
   gagggtattt tatgaggaat ctaacccagc tcaatatgta tggggttccg ccccactcgg  300
   catgtaggca ggaactacag cggtcttgtg ggctaccatg gaacgaaaga cagtattaaa  360
   acatatgtgc gaaatacaac tttagttcgt agacataaag acggtaatgc agtaatgtgc  420
   cactgatgag cactcgccgg aacatggcta cgtcggtatc atttggaagc tgtttttcat  480
   gtccaaatat gaaggcaaag taaaatatac ttccgcgacc ataaagagga aaagaaaagt  540
   gcgcctcgta ttgtagatgg taaaacacgt cttttcataa tatgtactgc actgattgtt  600
   ggagtatacg tttgcaacac gtgacaccat ttacaacaaa tactaaaaag agcactttta  660
   ttgtgctaac acaaaaaaaa aacacaagag ttcagaaaac agacaagaaa aagcaaaaga  720
   cgtctaaata acatacaaat ccagatgtca gacgcaaaat acaaattacg gtgttacagg  780
   tgatatctca cgtctgcgag ataaagcagt tgttttactg caaacgaagg taataccagt  840
   ggcgtagact agagcctcta gcttcgagag ggatcgcgaa gtattgggac tacctcacgc  900
   caccggtaag ggcatcggtg cagcgagagg ttatcgccca atacaacaac aatgataatg  960
   acgtgcaagc agataatagt gaaaaaataa cagatactag agtataaaaa ggggatgctg 1020
   ggagtggaca ggcacagtcg tggtgtggca gcaaaca                          1057
<210> SEQ ID NO 2
   <211> 1041
   <212> DNA
   <213> Trichoplusia ni
<220>
   <223> Promotor pB2
<400>
   cttgaatgtt agtgaaaccc cctgcgacac aagtattaca ttccttagtg cttgaatcct   60
   ttaggaaaga aaagccaatt ttcaaaatct tagcacttgt taactcgcga aaaagaccaa  120
   cagatttccc atactacaat tcgacattag aaatgtaaac ccattatcat tatttacgcc  180
   tcatttccat ccaataataa gtttaagtac gttgagataa aactggctta cctagaactt  240
   gacatggcga cctcttgcac tctgtatctc aagtcaactt tctctatcca aatatttgat  300
   aacatttgac atgatattga agtaagattg ttactaaggc ttacattgta atattactga  360
   cgcaagttct ttatcaataa aatagctgaa aacaaaaaaa aaaacatcga ttagggtgac  420
   tgaaggttac attggggtag gttatggtta atacgtaatg gtttaacacc aaaacgatat  480
   catggattga ctttataaat tttatataag gtgtaataat atttttaatg agtggacgcg  540
   tcgggtcaat gtcctgccta ttgacgtcat aacatattag gtgattatat taaaaatact  600
   caaatattac ttgcaagttt aagtttcatc ataatctgat cataagtttc acccaaacag  660
   aaaccaaaag cataactatc tgctatttga atatctttag cttcccatga agaaagatta  720
   ccgtaaccat cactaggatt ttatacgatt gtagaaaata aagtattctc agtctctttt  780
   cagtttaaaa tctgctggca tttttacaag tcgctgtatc agtcaatgtt tatacaatat  840
   gtcaatgtac tttcgtatta atcagaaaaa aatattctac tagttttgat aagctatcac  900
   ttttgttaca ttgtactgcc ctttacagtt catcaggtat ttatgaatga catattggag  960
   aaacatcgta atcagtccag tataaaaagg ggtgcattct cggtaagagt acagttgaac 1020
   tcacatcgag ttaactccac g                                          1041
<210> SEQ ID NO 3
   <211> 19
   <212> DNA
   <213> Trichoplusia ni
<220>
   <223> Primer pBJHSP1-Fwd: 5'... 3'
<400>
   aaaatgtaca aagattcgc 19
<210> SEQ ID NO 4
   <211> 18
   <212> DNA
   <213> Trichoplusia ni
<220>
   <223> Primer pBJHSP1-Rev: 5'... 3'
<400>
<210> SEQ ID NO 5
   <211> 21
   <212> DNA
   <213> Trichoplusia ni
<220>
   <223> Primer pBJHSP2-Fwd: 5'... 3'
<400>
   cttgaatgtt agtgaaaccc c 21
<210> SEQ ID NO 6
   <211> 20
   <212> DNA
   <213> Trichoplusia ni
<220>
   <223> Primer pBJHSP2-Rev: 5'... 3'
<400>
   cacatcgagt taactccacg 20